(19) Europäisches Patentamt
European Patent Office
Office européen des brevets

(11) Publication number : **0 466 365 A2**

(12) # EUROPEAN PATENT APPLICATION

(21) Application number : **91305817.8**

(22) Date of filing : **27.06.91**

(51) Int. Cl.$^5$ : **C07H 17/08,** A61K 31/70, C12P 21/00

The applicant has filed a statement in accordance with Rule 28 (4) EPC (issue of a sample only to an expert). Accession number(s) of the deposit(s): ATCC 55060.

(30) Priority : **03.07.90 US 547269**

(43) Date of publication of application : **15.01.92 Bulletin 92/03**

(84) Designated Contracting States : **CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Petuch, Brian R.**
**41-2 Carriage Stop**
**Florence, NJ 08518 (US)**
Inventor : **Arison, Byron H.**
**88 Century Lane**
**Watchung, NJ 07060 (US)**
Inventor : **Chen, Shieh-Shung Tom**
**12 Scott Drive**
**Morganville, NJ 07751 (US)**

(74) Representative : **Thompson, John Dr. et al**
**Merck & Co., Inc. European Patent Department Terlings Park Eastwick Road**
**Harlow, Essex CM20 2QR (GB)**

(54) **Novel immunosuppressant fermentation products of a microorganism.**

(57) Fermentation of a nutrient medium comprising immunomycin as a substrate by a Bacillus subtillus provides novel compounds which are highly potent immunosuppressive agents.

EP 0 466 365 A2

FIG. 1

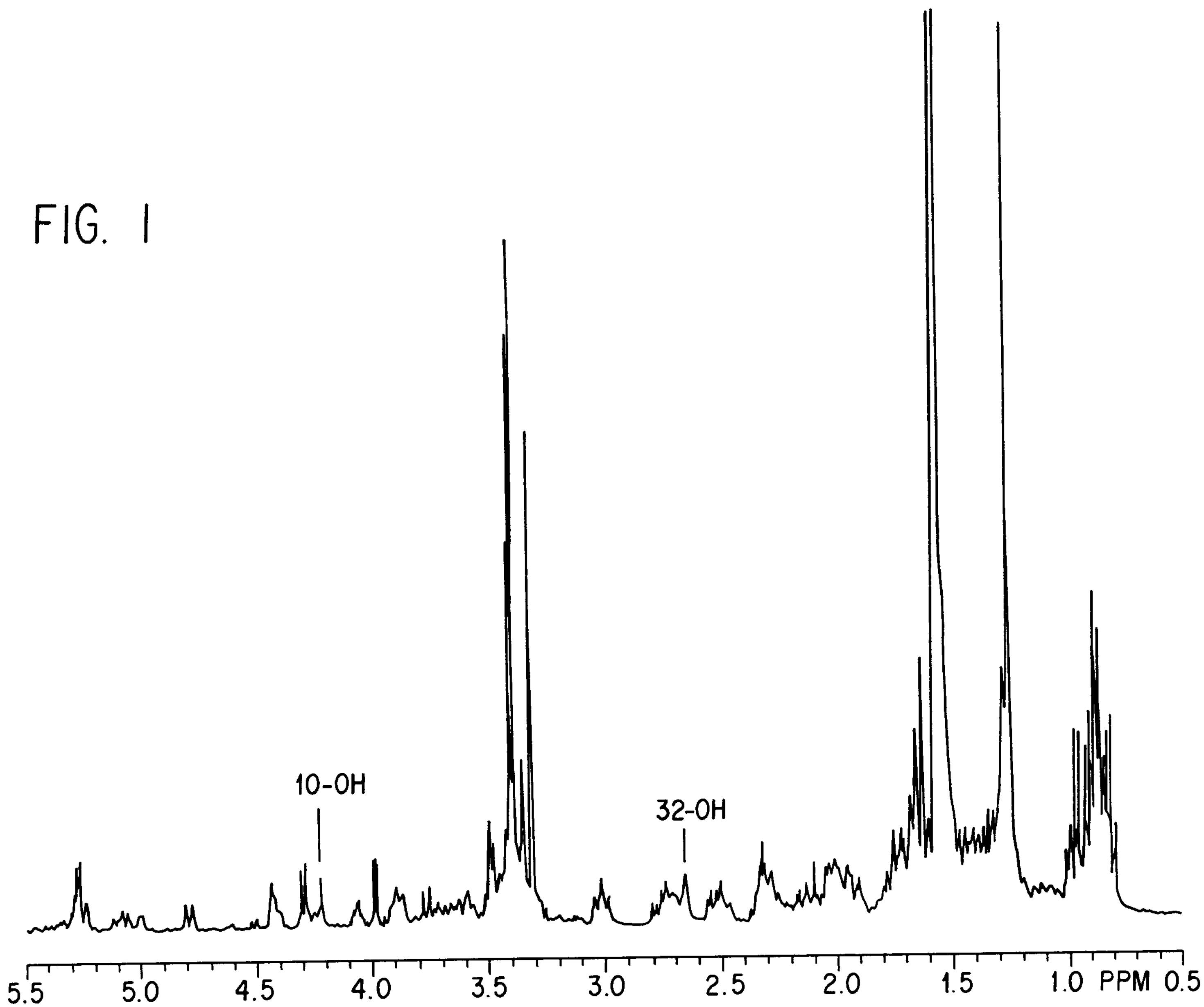

## BACKGROUND OF THE INVENTION

In 1983 the US FDA licensed cyclosporin, an extremely effective anti-rejection drug that revolutionized the field of organ transplant sugery. The drug acts by inhibiting the body's immune system from mobilizing its vast arsenal of natural protecting agents to reject the transplant's foreign protein.

As effective as the drug is in fighting transplantation rejection, it suffers drawbacks in causing liver damage, kidney failure and ulcers which in many cases can be very severe.

A new macrolide immunosuppressant, FK-506, which is reputed to be 100 times more effective than cyclosporin was disclosed by H. Tanaka et al., in EPO Publ. No. 0184162. The same reference also discloses related compounds, including FK-520, also known by the common name immunomycin.

The novel compounds of this invention are related to immunomycin. Immunomycin and related compounds are also disclosed by M. E. Cooper et al., in EPO Publ. No. 323042. However the instant compounds possess significant structural features which readily differentiate them from the prior art compounds.

## Brief Description of the Figures

Figure 1 is an 1H nuclear magnetic resonance (NMR) spectrum taken at 400 MHz of L-686,977, a compound of the instant invention described hereinbelow as an Example.

Figure 2 is an NMR spectrum taken at 400 MHz of immunomycin, which has been given the numerical descriptor L-683,590.

Figure 3 is an NMR spectrum taken at 400 MHz of a chemical degradative residue of L-686,977 and a NMR spectrum of glucose included for comparison.

## SUMMARY OF THE INVENTION

This invention is concerned with a series of novel macrocyclic lactones which are produced by the fermentation, with immunomycin as the substrate, of a nutrient medium with a strain of the microorganism <u>Bacillus subtilis</u> MB-4974 which is a known microorganism available from the American Type Culture Collection as ATCC 55060. Thus, it is an object of this invention to provide such novel compounds, and a method for preparing such products microbiologically. It is a further object of this invention to provide processes for the recovery and purification of the compounds from the fermentation broth. These substances have immunosuppressant activity. Further objects of this invention will become apparent from the following description of this invention.

## DESCRIPTION OF THE INVENTION

This invention is concerned with a series of novel macrocyclic lactones having the formula:

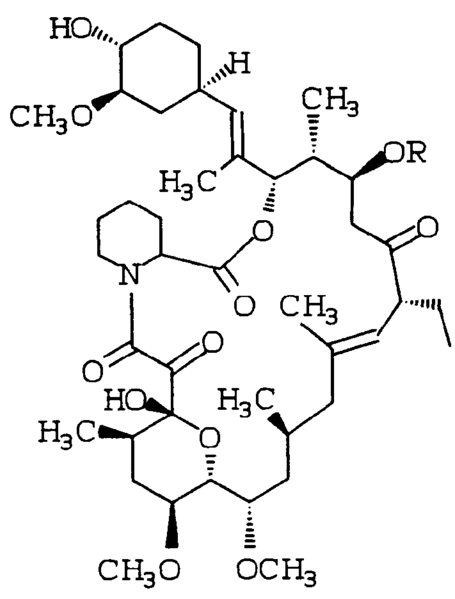

wherein R is sugar residue.

The term "sugar residue" refers to a polyhydroxy 5- or 6-membered cyclic acetal which is optionally substituted on the ring carbon atoms with lower alkyl and in which the hydroxy groups may be optionally substituted with lower alkyl or lower alkanoyl. The preferred sugar residues are glucopyranosyl, galactopyranosyl, ribofuranosyl, arabinofuranosyl, mannopyranosyl ,xylofuranosyl and the like.

In accordance with this invention, a series of novel compounds are described, which are prepared by growing, under controlled conditions with the macrolactone immunomycin as a substrate, a known strain of microorganism, Bacillus subtilis sub. sp. MB-4974. The compounds are obtained by fermentation and recovered in substantially pure form as described herein.

The culture designated MB-4974 is in the culture collection of Merck & Co., Inc., Rahway, N.J. A sample of this culture, capable of producing the herein described compound, is available in the permanent culture collection of the American Type Culture Collection at 12301 Parklawn Drive, Rockville, Md. 20852, and has been assigned the accession number ATCC 55060.

The instant compounds are produced from immunomycin during the aerobic fermentation of suitable aqueous nutrient media under conditions described hereinafter, with a strain of Bacillus subtilis MB-4974. Aqueous media such as those used for the production of many antibiotic substances are suitable for use in this process for the production of these macrocyclic compounds. Such nutrient media contain sources of carbon and nitrogen assimilable by the microorganism and generally low levels of inorganic salts. In addition, the fermentation media may contain traces of metals necessary for the growth of the microorganisms, and production of the desired compounds. These are usually present in sufficient concentrations in the complex sources of carbon and nitrogen, which may be used as nutrient sources, but can, of course, be added separately to the medium if desired.

In general, carbohydrates such as sugars, for example dextrose, sucrose, maltose, lactose, dextran, cerelose, corn meal, oat flour, and the like, and starches are suitable sources of assimilable carbon in the nutrient media. The exact quantity of the carbon source which is utilized in the medium will depend, in part, upon the other ingredients in the medium, but it is usually found that an amount of carbohydrate between 0.5 and 5% by weight of the medium is satisfactory. These carbon sources can be used individually or several such carbon sources may be combined in the same medium.

Various nitrogen sources such as yeast hydrolysates, yeast autolysates, yeast cells, tomato paste, corn meal, oat flour, soybean meal, casein hydrolysates, yeast extracts, corn steep liquors, distillers solubles, cottonseed meal, meat extract and the like, are readily assimilable by Bacillus subtilis MB-4974 in the production of the instant compounds. The various sources of nitrogen can be used alone or in combination in amounts ranging from 0.2 to 6% by weight of the medium.

Among the nutrient inorganic salts, which can be incorporated in the culture media are the customary salts capable of yielding sodium, potassium, magnesium, ammonium, calcium, phosphate, sulfate, chloride, carbonate, and like ions. Also included are trace metals such as cobalt, manganese, and the like.

It should be noted that the media described hereinbelow and in the Examples are merely illustrative of the wide variety of media, which may be employed, and are not intended to be limitative.

The following are Examples of media suitable for growing strains of Bacillus subtilis MB-4974.

Medium 1

| | |
|---|---|
| Dextrose | 1.0 g |
| Dextrin (Fisher) | 10.0 g |
| Beef Extract (Difco) | 3.0 g |
| Yeast Autolysate (Ardamine pH, Yeast Prod.) | 5.0 g |
| NZ Amine Type E (Sheffield) | 5.0 g |
| $MgSO_4.7H_2O$ | 0.05 g |
| Phosphate Buffer | 2 ml |
| $CaCO_3$ | 0.5 g |
| $dH_2O$ | 1000 ml |

pH 7.0-7.2

| Phosphate Buffer: | | |
|---|---|---|
| | $KH_2PO_4$ | 91.0 g |
| | $Na_2HPO_4$ | 95.0 g |
| | $dH_2O$ | 1000 ml |
| | pH 7.0 | |

Medium 2

| | |
|---|---|
| Yeast Extract (Difco) | 4.0 g |
| Malt Extract (Difco) | 10.0 g |
| Dextrose | 4.0 g |
| $dH_2O$ | 1000 ml |
| Agar | 20 g |

pH 7.2

Medium 3

Basal

| | |
|---|---|
| Sucrose | 103 g |
| $K_2SO_4$ | 0.25 g |
| Glucose | 10 g |
| L-Asparagine | 1.8 g |
| Casamino Acids (Difco) | 0.1 g |
| $MgCl_2.6H_2O$ | 10.12 g |
| Trace Element Mix A | 2 ml |
| $dH_2O$ | to 700 ml |
| Agar | 22.0 g |

Post-sterilization additions, per 700 ml Basal:

100 ml of $CaCl_2$ solution (29.5 g/1000 ml $dH_2O$)
100 ml of $KH_2PO_4$ solution (0.5 g/1000 ml $dH_2O$)
100 ml of Tes solution (0.3 g Tris HCl + 0.1 g EDTA + 0.14 g NaCl in 1000 ml $dH_2O$, adjust to pH 8.0)

Trace Element Mix A Composition:

| | |
|---|---|
| $Fe(SO_4)_3.7H_2O$ | 250 mg |
| $MnCl_2.4H_2O$ | 500 mg |
| $CuCl_2.2H_2O$ | 25 mg |
| $CaCl_2.2H_2O$ | 1000 mg |
| $H_3BO_3$ | 50 mg |
| $(NH_4)_6Mo_7O_{24}.4H_2O$ | 20 mg |
| $ZnSO_4.7H_2O$ | 100 mg |
| $Co(NO_3)_2.6H_2O$ | 20 mg |
| 0.1N HCl | 1000 ml |

Medium 4

| | |
|---|---|
| Dextrose | 20 g |
| Soya meal | 5 g |
| Yeast Extract (Fidco) | 5 g |
| NaCl | 5 g |
| MES buffer | 9.8 g |
| $dH_2O$ | 1000 ml |
| pH 7.0 | |

Medium 5

| | |
|---|---|
| Dextrose | 45 g |
| Peptonized Milk (Sheffield) | 24 g |
| Ardamine pH (Yeast Products, Inc.) | 2.5 g |
| Polyglycol 2000 (Dow) | 2.5 ml |
| $d/H_2O$ | 1000 ml |

pH 7.0

Medium 6

| | |
|---|---|
| Dextrose | 2.0% |
| Yeast Extract (Difco) | 2.0 |
| Casamino Acids (Difco) | 2.0 |
| $KNO_3$ | 0.2 |
| $MgSO_4.7H_2O$ | 0.05 |
| NaCl | 0.05 |
| $FeSO_4.7H_2O$ | 0.0025 |
| $CaCl_2.2H_2O$ | 0.002 |
| $ZnSO_4.7H_2O$ | 0.001 |
| $MnSO_4.H_2O$ | 0.0005 |
| d $H_2O$ | 1000 ml |

pH 7.0 with NaOH

Medium 7

| | |
|---|---|
| Dextrose | 0.1% |
| Soluble Starch (Fisher) | 1.0 |
| Beef Extract (Difco) | 0.3 |
| Yeast Autolysate (Ardamine pH Yeast Products) | 0.5 |
| NZ Amine Type E (Sheffield) | 0.5 |
| $MgSO_4.7H_2O$ | 0.005 |
| $KH_2PO_4$ | 0.0182 |
| $Na_2HPO_4$ | 0.0190 |
| $CaCO_3$* | 0.05 |
| d $H_2O$ | 1000 ml |

pH 7.0-7.2 with NaOH

* Added after pH adjustment

The fermentation employing Bacillus subtilis MB-4974 can be conducted at temperatures ranging from

about 20°C to about 40°C. For optimum results, it is most convenient to conduct these fermentations at a temperature in the range of from about 24°C to about 30°C. Temperatures of about 27°-28°C are most preferred. The pH of the nutrient medium suitable for producing the instant compounds can vary from about 5.0 to 8.5 with a preferred range of from about 6.0 to 7.5.

Small scale fermentations are conveniently carried out by placing suitable quantities of nutrient media in a flask employing known sterile techniques, inoculating the flask with either spores or vegetative cellular growth of Bacillus subtilis MB-4974 loosely stoppering the flask with cotton and permitting the fermentation to proceed in a constant temperature room of about 28°C on a rotary shaker at from 95 to 300 rpm for about 2 to 10 days. For larger scale work, it is preferable to conduct the fermentation in suitable tanks provided with an agitator and a means of aerating the fermentation medium. The nutrient medium is made up in the tank and after sterilization is inoculated with a source of vegetative cellular growth of Bacillus subtilis MA-4974. The fermentation is allowed to continue for from 1 to 8 days while agitating and/or aerating the nutrient medium at a temperature in the range of from about 24° to 37°C. The degree of aeration is dependent upon several factors such as the size of the fermentor, agitation speed, and the like. Generally the larger scale fermentations are agitated at about 95 to 500 RPM and about 2 to 20 cubic feet per minute (CFM) of air.

The separation of the novel compounds from the whole fermentation broth and the recovery of said compounds is carried out by solvent extraction and application of chromatographic fractionations with various chromatographic techniques and solvent systems.

The instant compounds have slight solubility in water, but are soluble in organic solvents. This property may be conveniently employed to recover the compounds from the fermentation broth. Thus, in one recovery method, the whole fermentation broth is combined with approximately an equal volume of an organic solvent. While any organic solvent may be employed, it is preferable to use a water immiscible solvent such as ethyl acetate, methylene chloride, chloroform and the like. Generally several extractions are desirable to achieve maximum recovery. The solvent removes the instant compounds as well as other substances lacking the immunosuppressive activity of the instant compounds. If the solvent is a water immiscible one, the layers are separated and the organic solvent is evaporated under reduced pressure. If the solvent is water miscible, it can be extracted with a water immiscible solvent to separate the entrained water. This solvent can then be concentrated under reduced pressure. The residue is placed onto a chromatography column containing preferably, silica gel. The column retains the desired products and some impurities, but lets many of the impurities, particularly the nonpolar impurities, pass through. The column is washed with a moderately polar organic solvent such as methylene chloride or chloroform to further remove impurities, and is then washed with a mixture of methylene chloride or chloroform and an organic solvent of which acetone, ethyl acetate, methanol, and ethanol and the like are preferred. The solvent is evaporated and the residue further chromatographed using column chromatography, thin layer chromatography, preparative layer chromatography, high pressure liquid chromatography preferably reverse phase, and the like, with silica gel, aluminum oxide, dextran gels and the like, as the chromatographic medium, with various solvents and combinations of solvents as the eluent. Thin layer, high pressure, liquid and preparative layer chromatography may be employed to detect the presence of, and to isolate the instant compound. The use of the foregoing techniques as well as others known to those skilled in the art, will afford purified compositions containing the instant compound. The presence of the desired compound is determined by analyzing the various chromatographic fractions for biological activity against selected parasites, or physicochemical characteristics. The structure of the instant compounds has been determined by detailed analysis of the various spectral characteristics of the compounds, in particular their nuclear magnetic resonance, mass, ultraviolet and infrared spectra.

Suitable formulations of the material may also include conventional pharmaceutically acceptable biolabile esters of the compound of the instant invention formed via the hydroxy groups on the molecule, such as the acetate.

The compounds of the instant invention possess pharmacological activity such as immunosuppressive activity, antimicrobial activity, and the like, and therefore are useful for the treatment and prevention of the transplantation rejection of organs or tissues such as heart, kidney, liver, medulla ossium, skin, etc., graft-versus-host diseases by medulla ossium transplantation, auto-immune diseases such as rheumatoid arthritis, systemic lupus erythematosus, Hashimoto's thyroiditis, multiple sclerosis, myasthenia gravis, type I diabetes, uveitis, and the like.

The pharmaceutical composition of this invention can be used in the form of a pharmaceutical preparation, for example, in solid, semisolid or liquid form, which contains the compound of the present invention as an active ingredient, in admixture with an organic or inorganic carrier or excipient suitable for external, enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used are water, glucose, lactose, gum

acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The active object compound is included in the pharmaceutical composition in an amount sufficient to produce the desired effect upon the process or condition of diseases.

For applying this composition to a human, it is preferable to apply it by parenteral or enteral administration. While the dosage of therapeutically effective amount of the compound of the instant invention varies from, and also depends upon, the age and condition of each individual patient to be treated, a daily dose (calculated on the basis of a 70 kg man) of about 0.01-1000 mg, preferably 0.1-500 mg and more preferably 0.5-100 mg, of the active ingredient is generally given for treating diseases, and an average single dose of about 0.5 mg, 1 mg, 5 mg, 10 mg, 50 mg, 100 mg, 250 mg and 500 mg is generally administered.

The invention is further defined by reference to the following example, which is intended to be illustrative and not limiting.

## EXAMPLE

## Transformation Methodology

### Transformation Method I: Resting Cell Biotransformation

**Media:** Seed Medium A

| | g/l |
|---|---|
| Dextrose | 20.0 g |
| Soya meal | 5.0 g |
| Yeast Extract | 5.0 g |
| NaCl | 5.0 g |
| MES buffer | 9.8 g |
| 1000 ml distilled $H_2O$ pH 7.0 | |

Transformation Medium B

| | |
|---|---|
| Sterile glucose | 10.0 g |
| Immunomycin in 0.2 ml DMSO | 200.0 mg |
| 1000 ml 0.1M MES buffer pH 6.0 | |

A lyophile tube was aseptically opened and grown in seed Medium A (50 ml in a 250 ml unbaffled Erlenmyer flask) for 48 hours on a rotary shaker (220 rpm) at 29.5°C.

This seed (5 ml) was then used to inoculate second stage flasks (Medium A, 50 ml in a 250 ml unbaffled Erlenmeyer flask containing 20 μg/ml immunomycin as inducer) for 24 hours on rotary shaker (220 rpm) at 29.5°C.

The resting cells were prepared by pelleting the cells from the second stage flasks using centrifugation, washing once with sterile saline and resuspending the cells in Medium B. The cultures were then incubated for 18 hours at 29.5°C on a rotary shaker.

Transformation Method II: Alginate Immobilized Cell Biotransformation

Media: Seed Medium A: same as in Method 1

Transformation Medium C

| | |
|---|---|
| Sterile glucose | 10.0 g |
| Immunomycin in 0.2 ml DMSO | 2.0 mg |
| 10 ml 0.1M MES buffer/0.1M $CaCl_2$ in $H_2O$ pH 6.0 | |

The pellets from the second stage flasks described in Method I above were washed twice with saline and 9.4 g (wet weight) of the cells was resuspended in 10 ml of saline. A solution of 0.75 mg of Kelco LV alginic acid in 40 ml of saline was added to the cell mixture and the mixture then slowly dripped into ice cold 0.1M $CaCl_2$. The mixture was allowed to harden for 15 minutes and then the beads were collected by filtration thru cheesecloth. Two grams of beads were incubated in 10 ml of Medium C at 29.5°C for 18 hours.

Following incubation, the whole broths were extracted as follows:

Extraction Methodology

a. 150 ml of whole broth was extracted with three 75 mL portions of methylene chloride.

b. The pooled methylene chloride extracts were dried with anhydrous sodium sulfate, then filtered and concentrated under vacuum to yield a brown oil.

c. The oil was washed 2 times with cold petroleum ether and again dried under vacuum.

d. The oil was dissolved in a acetonitrile:water mixture (45% acetonitrile) with 0.1% $H_3PO_4$ and purified by high performance liquid chromatograhy (Whatman Partisil 10 ODS-3; flow rate 4.9 ml/min.; acetonitrile:water + 0.1% $H_3PO_4$ gradient of 45% to 80% acetonitrile over 20 mins.) at 57°C.

e. The fractions at retention time 15.6 min.were pooled and the pH adjusted to 7.0. The mixture was then concentrated under vacuum to an aqueous residue which was then extracted 2 times with methylene chloride.

f. The organic extracts were pooled and then washed 2 times with water. The organic solution was then concetrated under vacuum at 40°Cto provide the compound of the instant invention which was given the label L-686,977.

The compound from Example 1 was heated in anhydrous trifluoroacetic acid (TFA) at 37oC for 15 mins. The reaction mixture was then concentrated to dryness under vacuum and the residue was dissolved in chloroform. The organic solution was extracted 2 times with water and the aqueous extracts were combined and then concentrated under vacuum at 50°C. The residue was dissolved in water and purified by medium pressure liquid chromatography (C-18 SepPak). The aqueous solution was then concentrated and purified with high performence liquid chromatograhy (Brownlee amino bonded-phase HPLC column; 90% aqueous acetonitrile) to provide the sugar residue of the compound of the instant invention.

Based on the following analytical data, the instant compound of the Example is believed to have the following structural formula:

M.S. (FAB) m/e= 953 ($M^+$);

The nuclear magnetic resonance (NMR) spectrum for this compound is found in the attached Figures 1 and was originally recorded in $CDCl_3$ at ambient temperature on a Varian XL-400 NMR Spectrometer. Also attached (Figure 2), for comparative purposes, is the NMR spectrum of immunomycin. Chemical shifts are shown in ppm relative to tetramethylsilane as an internal standard at zero ppm.

The NMR spectrum of the cleaved sugar residue (R in the structure below), which was isolated as described hereinabove, is found in the attached Figure 3, which also contains the n.m.r. of authentic glucose. Comparison of the two spectra show that the group R in the structure is glucose having the structure shown below.

The structure is as follows:

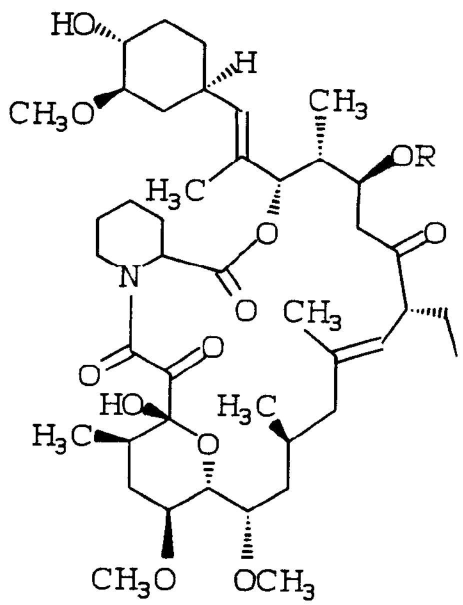

R=

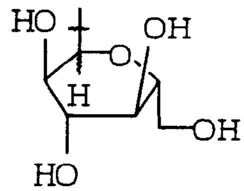

## Claims

1. A compound having the formula:

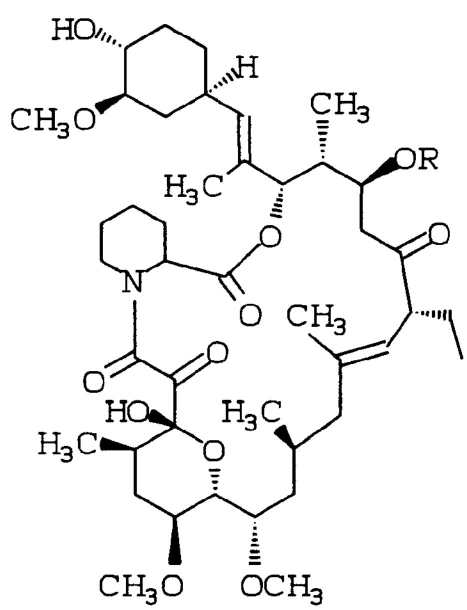

wherein R is a sugar residue.

2. The compound of Claim 1 wherein R is:
   a) glucopyranosyl,
   b) galactopyranosyl,
   c) glucanopyranosyl,
   d) ribofuranosyl,
   e) arabinofuranosyl,
   f) mannopyranosyl,

or
g) xylofuranosyl

3. The compound of Claim 1 wherein R is:

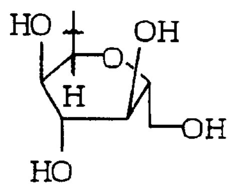

4. A pharmaceutical composition comprising a therapeutically effective amount of the compound of Claim 1 and a pharmaceutically acceptable, substantially non-toxic carrier or excipient.

5. A pharmaceutical composition comprising a therapeutically effective amount of the compound of Claim 2 and a pharmaceutically acceptable, substantially non-toxic carrier or excipient.

6. A pharmaceutical composition comprising a therapeutically effective amount of the compound of Claim 3 and a pharmaceutically acceptable, substantially non-toxic carrier or excipient.

7. The use of the compound of Claim 1 for the manufacture of a medicament for treating a human host to prevent transplantation rejection, or for treating autoimmune disease or infectious disease.

8. The use of the compound of Claim 2 for the manufacture of a medicament for treating a human host to prevent transplantation rejection, or for treating autoimmune disease or infectious disease.

9. The use of the compound of Claim 3 for the manufacture of a medicament for treating a human host to prevent transplantation rejection, or for treating autoimmune disease or infectious disease.

FIG. 1

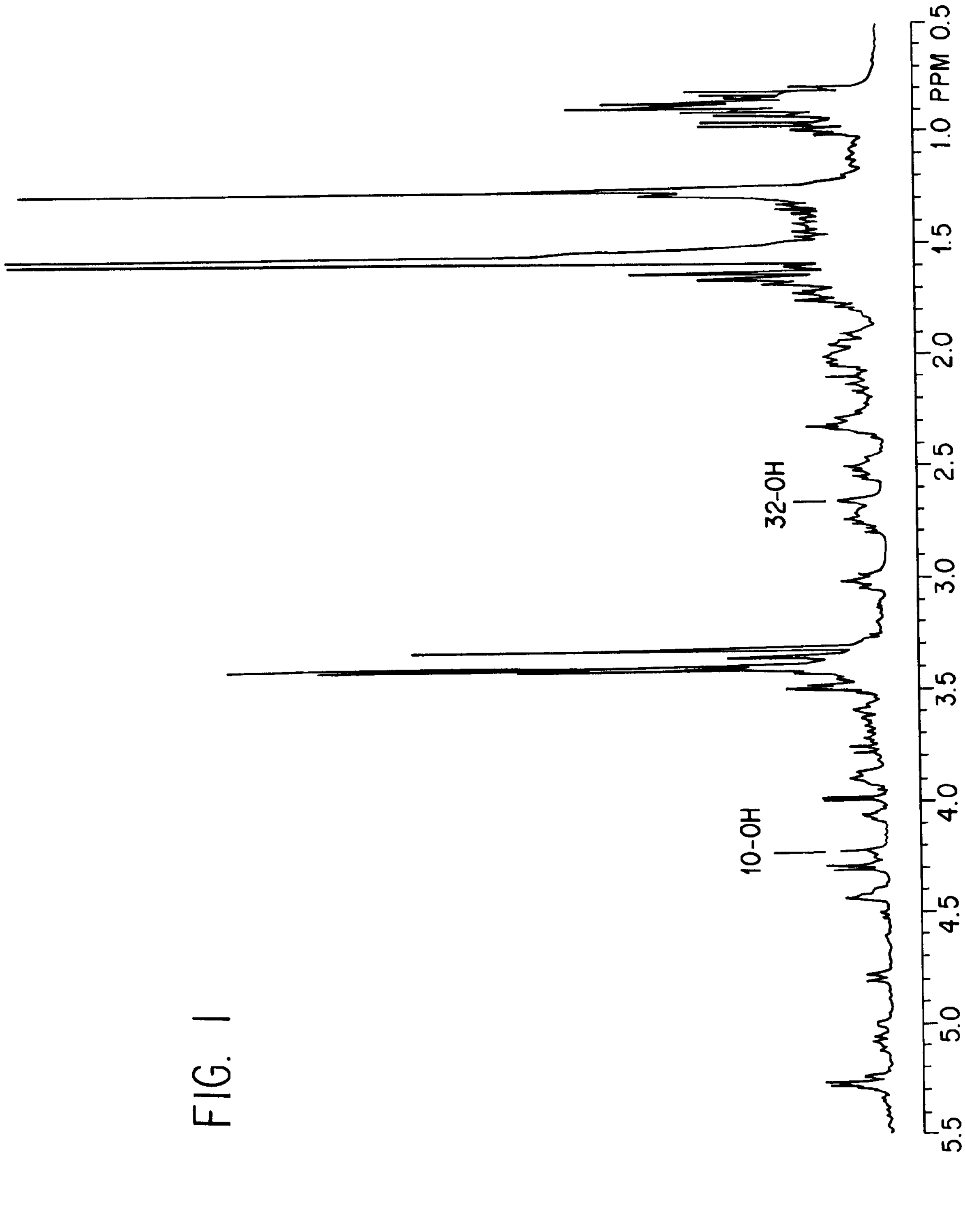

FIG. 2

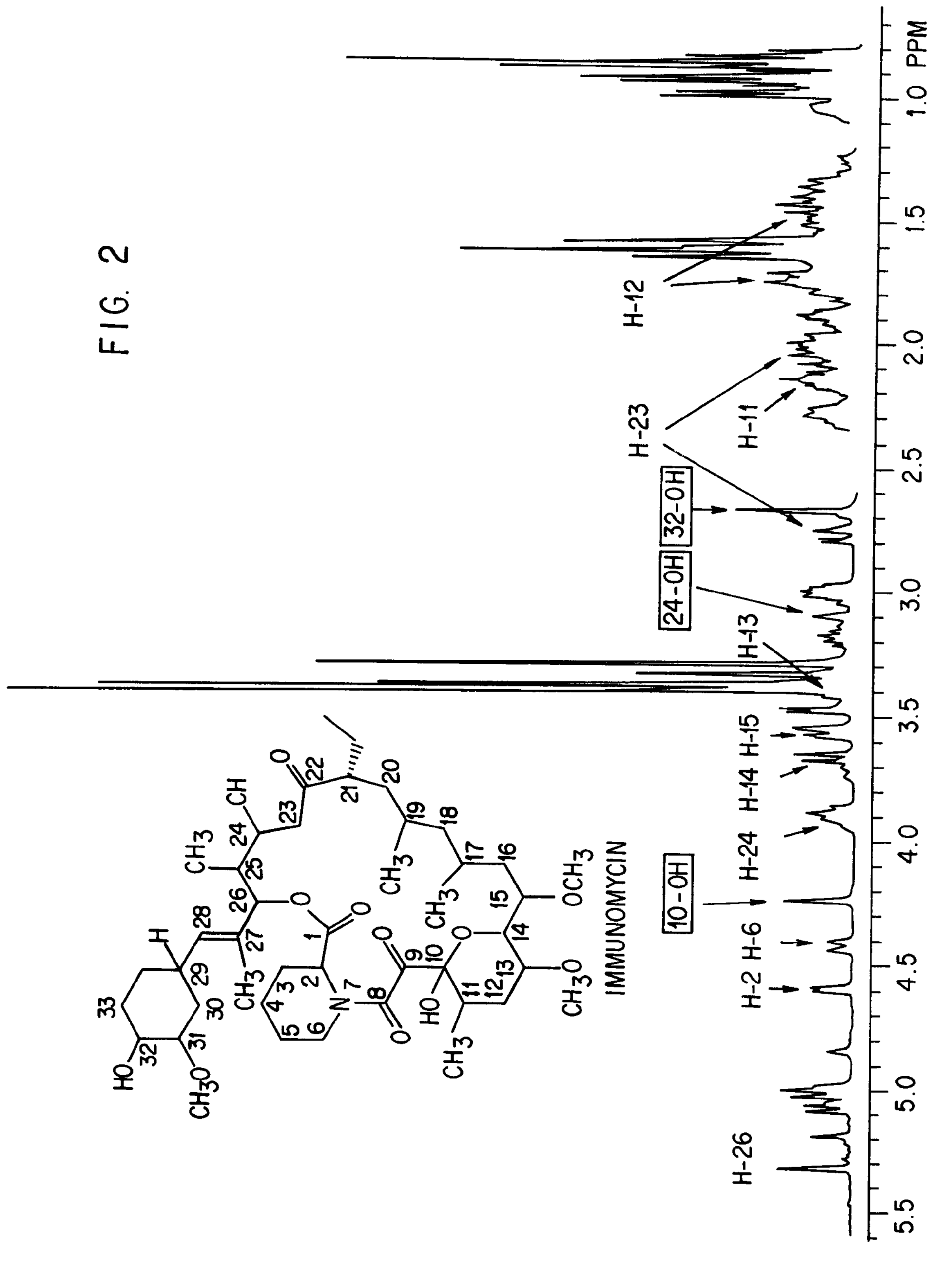


IMMUNOMYCIN

FIG. 3

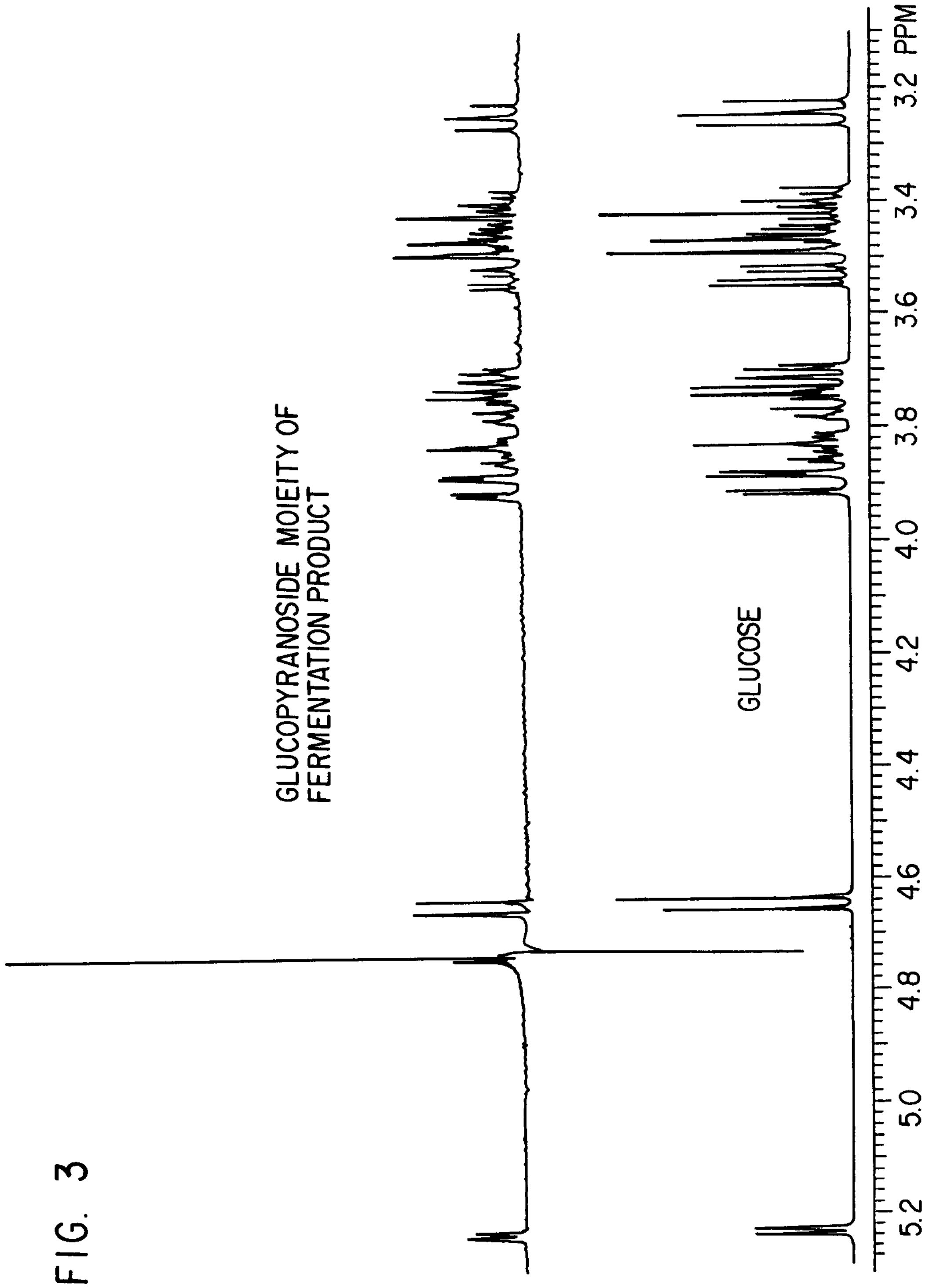
GLUCOPYRANOSIDE MOIEITY OF
FERMENTATION PRODUCT
GLUCOSE
5.2
5.0
4.8
4.6
4.4
4.2
4.0
3.8
3.6
3.4
3.2 PPM